# EUROPEAN PATENT APPLICATION

(11) **EP 1 702 571 A1**
(43) Date of publication of application: **20.09.2006**
(21) Application number: 06005398.0
(22) Date of filing: 16.03.2006
(51) Int. Cl.: A61B 17/11, A61B 17/00, A61M 29/02, A61M 25/10, A61B 19/00

(54) **A device for extending a distal end of an anatomic tube**

(30) Priority: 17.03.2005 US 82625
(71) Applicant: The Intertech Group, Inc., North Charleston, SC 29405 (US)
(72) Inventor: Zucker, Jerry, Charlston South Carolina 29407 (US)
(74) Representative: Luderschmidt, Schüler & Partner

(57) **Abstract**

A device and a method for extending a distal end of an anatomic tube. This device (10) includes an accordion pleated bladder (12), which includes a proximal end (14) and a distal end (16). The method includes the steps of (1)providing a device (10) for extending a distal end of an anatomic tube including an accordion pleated bladder (12), wherein said bladder has a proximal end (14) and a distal end (16); (2)placing the device (10) for extending a distal end of an anatomic tube in the anatomic tube; (3)connecting the device (10) to a pressure generator; (4)increasing the internal pressure of the device (10) thereby extending the bladder (12) along its longitudinal axis; and (5)thereby extending the anatomic tube along its longitudinal axis.

## Description

### Field of Invention

The instant application relates to a device, and a method for extending a distal end of an anatomic tube.

### Background of the Invention

Congenital abnormalities may cause serious threats to the well-being of individuals with such abnormalities. Congenital abnormalities, i.e. birth defects, include a wide range of malformations that occur during the fetal development. For example, esophageal atersia is a congenital abnormality where the esophagus fails to connect to the stomach. As a result, the esophagus ends in a pouch, and nothing the baby swallows gets into the stomach.

In the case of a baby with esophageal atersia, a surgery is generally required to connect the esophagus to the stomach. In general, a residual esophagus is expanded via a bladder thereby facilitating the connection between the esophagus and the stomach via surgery. Residual esophagus, as used herein, may be a tubular extension of the stomach; or in the alternative, it may be an incomplete esophagus that fails to connect to the stomach. However, the expansion of the residual esophagus via the current methods causes expansion of the residual esophagus both circumferentially and longitudinally. Although the longitudinal expansion of the residual esophagus facilitates the connection to the stomach and the esophagus; thus a desired outcome, the circumferential expansion of the residual esophagus is an undesired by-product, which must be corrected via surgery.

Accordingly, there is a need for a device to facilitate the extension of the distal end of an anatomic tube, e.g. a residual esophagus, along its longitudinal axis while minimizing any circumferential expansion.

### Summary of the Invention

The instant application relates to a device, and a method for extending a distal end of an anatomic tube. This device includes an accordion pleated bladder, which includes a proximal end and a distal end. The method includes the steps of (1)providing a device for extending a distal end of an anatomic tube including an accordion pleated bladder, wherein said bladder has a proximal end and a distal end; (2)placing the device for extending a distal end of an anatomic tube in the anatomic tube; (3)connecting the device to a pressure generator; (4)increasing the internal pressure of the device thereby extending the bladder along its longitudinal axis; and (5)thereby extending the anatomic tube along its longitudinal axis.

### Brief Description of the Drawings

For the purpose of illustrating the invention, there is shown in the drawings a form that is presently preferred; it being understood, however, that this invention is not limited to the precise arrangements and instrumentalities shown.
Fig. 1 is a first embodiment of the instant invention in a compressed state; and
Fig. 2 is the first embodiment of the instant invention in an extended state.

### Detailed Description of the Invention

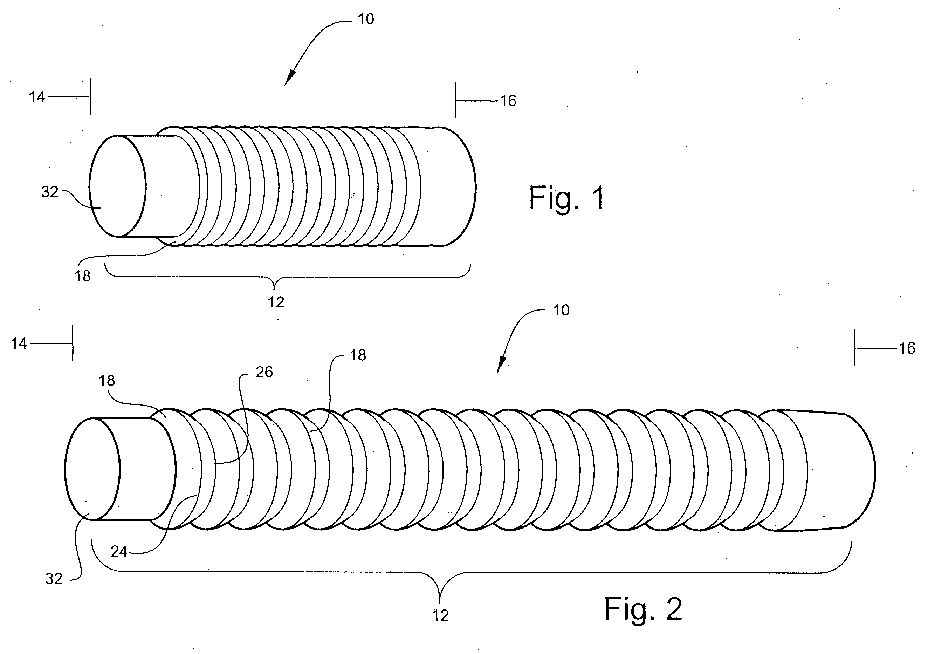

Referring to the drawings wherein like numerals indicate like elements, there is shown, in Figs. 1-2, a first embodiment of device 10 for extending a distal end of an anatomic tube such as a residual esophagus. Device 10 includes an accordion pleated bladder 12. The bladder 12 has a proximal end 14 and a distal end 16.

The instant invention, for convenience, has been described in terms of a device for extending the distal end of a residual esophagus; however, the instant invention is not so limited, and it may be employed to extend the distal end of any anatomic tube, e.g. the distal end of an intestine in the case of bowl removal. Residual esophagus, as used herein, may be a tubular extension of the stomach; or in the alternative, it may be an incomplete esophagus that fails to connect to the stomach.

Bladder 12 may be made of any biocompatible elastomeric material. For example, bladder 12 may be made of a biocompatible elastomeric material selected from the group consisting of silicone rubber, natural rubber, styrene-butadiene copolymer, polychloroprene, nitrile rubber, butyl rubber, polysulfide rubbercis-i,4-polyisoprene, ethylene-propylene terpolymers (EPDM rubber), certain metallocene grades of elasticated polyolefins such as elasticated polypropylene or elasticated polyethylene, and polyurethane rubber. Bladder 12 may have any shape adapted to facilitate the longitudinal extension of any anatomic tube such as a residual esophagus. Bladder 12 may, for example, have a tubular shape. Bladder 12 may further include different physical configurations; for example, bladder 12 may have a compressed state or an extended state, as shown in Figs. 1 and 2 respectively.

Bladder 12 is pleated. Bladder 12 may have any number of pleats 18. For example, bladder 12 may have a single pleat 18; or it may have a plurality of pleats 18. Pleats 18 may be any type of pleats; for example, pleats 18 may be annular pleats. Pleats 18 may have any shape; for example, pleats 18 may have a discoid shape.

Bladder 12 may further include a small diameter rib 24 or a large diameter rib 26. Bladder 12 may include any number of small diameter ribs 24 or any number of large diameter ribs 26. Bladder 12 may, for example, include one or a plurality of either of small diameter ribs 24 or large diameter ribs 26.

Small diameter rib 24 may have any shape adapted to minimize the uncontrolled circumferential expansion of bladder 18; for example, small diameter rib 24 may have an annular shape. Small diameter rib 24 may be made of any biocompatible elastomeric material. Small diameter rib 24, for example, may be a biocompatible elastomeric material selected from the group consisting of silicone rubber, natural rubber, styrene-butadiene copolymer, polychloroprene, nitrile rubber, butyl rubber, polysulfide rubbercis-i,4-polyisoprene, ethylene-propylene terpolymers (EPDM rubber), certain metallocene grades of elasticated polyolefins such as elasticated polypropylene or elasticated polyethylene, and polyurethane rubber. Small diameter rib 24 may be an integrated component of bladder 12, or in the alternative, it may be a separate component secured thereto bladder 12.

Large diameter rib 26 may have any adapted to minimize the uncontrolled circumferential expansion of bladder 12; for example, large diameter rib 26 may have an annular shape. Large diameter rib 26 may be made of any biocompatible elastomeric material. Large diameter rib 26 may, for example, may be a biocompatible elastomeric material selected from the group consisting of silicone rubber, natural rubber, styrene-butadiene copolymer, polychloroprene, nitrile rubber, butyl rubber, polysulfide rubbercis-i,4-polyisoprene, ethylene-propylene terpolymers (EPDM rubber), certain metallocene grades of elasticated polyolefins such as elasticated polypropylene or elasticated polyethylene, and polyurethane rubber. Large diameter rib 26 may be an integrated component of bladder 12, or in the alternative, it may be a separate component secured thereto bladder 12.

Proximal end 14 is a non-pleated proximal portion of bladder 12, and it may have a sealable opening 32. Proximal end 14 may include alternative means for securing bladder 12 to the proximal end of an anatomic tube such as a residual esophagus. Proximal end 14 may have any shape adapted to facilitate the extension of the distal end of an anatomic tube such a residual esophagus. For example, proximal end 14 may have a cylindrical shape adapted for securing bladder 12 to the proximal end of an anatomic tube such as a residual esophagus. Proximal end 14 may be adapted to expand circumferentially in a range of about 1% to about 30% thereby securing bladder 12 to the proximal end of an anatomic tube such as a residual esophagus. Proximal end 14 may further include first anchors (not shown) to secure the bladder 12 to the proximal end of an anatomic tube such as a residual esophagus. Proximal end 14 may further include the means for connecting bladder 12 to a pressure generator (not shown). Means for connecting bladder 12 to a pressure generator includes, but is not limited to, quick-connect fittings, threads, or other detachable coupling means such as clamps or fasteners.

Distal end 16 is a non-pleated distal portion of bladder 12, and it is sealed. Distal end 16 may include alternative means for securing bladder 12 to the distal end of an anatomic tube such as a residual esophagus. Distal end 16 may have any shape adapted to facilitate the extension of the distal end of an anatomic tube such a residual esophagus. For example, distal end 16 may have a cylindrical shape adapted for securing bladder 12 to the distal end of an anatomic tube such as a residual esophagus. Distal end 16 may be adapted to expand circumferentially in a range of about 1% to about 30% thereby securing bladder 12 to the distal end of an anatomic tube such as a residual esophagus. Distal end 16 may further include second anchors (not shown) to secure the bladder 12 to the distal end of an anatomic tube such as a residual esophagus.

In operation, device 10 is disposed in a residual esophagus, employing conventional methods known in the medical field. Device 10 is connected to a pressure generator (not shown) via a pressure pipe coupled with the proximal end 14. Pressure generator, for example, may generate pressure by pumping a fluid into device 10 thereby facilitating the expansion of bladder 12. Fluid, as used herein, refers to any liquid, e.g. saline solution, or any gas, e.g. CO₂. As the fluid is pumped into device 10, the internal pressure of device 10 increases thereby inducing bladder 12 to expand both circumferentially and longitudinally. However, the small diameter ribs 24 and the large diameter ribs 26 prevent the uncontrolled circumferential expansion of bladder 12 thereby facilitating the extension of the bladder 12 along its longitudinal axis. The controlled circumferential expansion of bladder 12 facilitates securing the proximal end 14 of the bladder 12 to the proximal end of the residual esophagus; and, it further facilitates securing the distal end 16 of bladder 18 to the distal end of the residual esophagus. The longitudinal extension of bladder 12 facilitates the extension of the distal end of the residual esophagus along its longitudinal axis. The extended residual esophagus may be maintained in an extended state until its extension along its longitudinal axis becomes permanent. For example, the extended residual esophagus may be maintained in an extended state for a period of about 1 month to about 6 months. During this period of extended state, as the residual esophagus gradually extends further, the internal pressure of device 10 declines; therefore, supplemental fluid is pumped into device 10 in order to increase the internal pressure of device 10 thereby inducing bladder 12 to further extend along its longitudinal axis. The longitudinal extension of bladder 12 facilitates further extension of the distal end of the previously extended residual esophagus. The addition of the supplemental fluid to facilitate further extension of the distal end of residual esophagus may be repeated as many times as necessary until an optimum extension of the distal end of the residual esophagus is achieved. In the alternative, the residual esophagus may be extended along its longitudinal axis as many times as necessary to induce a permanent extension along its longitudinal axis. For example the internal pressure of device 10 may, repeatedly, be reduced, and then, increased in order to induce a permanent extension along the longitudinal axis of the residual esophagus.

In an alternative operation, device 10 is disposed in residual esophagus, employing conventional methods known in the medical field. Device 10 is connected to a pressure generator (not Shown) via a pressure pipe coupled with the proximal end 14. Pressure generator, for example, may generate pressure by pumping a fluid into device 10 thereby facilitating the expansion of bladder 12. Fluid, as used herein, refers to any liquid, e.g. saline solution, or any gas, e.g. CO₂. The proximal end 14 of the bladder 12 is secured to the proximal end of the residual esophagus via first anchors, and the distal end 16 of the bladder 12 is secured to the distal end of the residual esophagus via second anchors. As the fluid is pumped into device 10, the internal pressure of device 10 increases, and the bladder 12 begins to expand both circumferentially and longitudinally. However, the small diameter ribs 24 and the large diameter ribs 26 prevent the uncontrolled circumferential expansion of bladder 12 thereby facilitating the extension of the bladder 12 along its longitudinal axis. The longitudinal extension of bladder 12 facilitates the extension of the distal end of the residual esophagus along its longitudinal axis. The extended residual esophagus may be maintained in an extended state until its extension along its longitudinal axis becomes permanent. For example, the extended residual esophagus may be maintained in an extended state for a period of about 1 month to about 6 months. During this period of extended state, as the residual esophagus gradually extends further, the internal pressure of device 10 declines; therefore, supplemental fluid is pumped into device 10 in order to increase the internal pressure of device 10 thereby inducing bladder 12 to further extend along its longitudinal axis. The longitudinal extension of bladder 12 facilitates further extension of the distal end of the previously extended residual esophagus. The addition of the supplemental fluid to facilitate further extension of the distal end of residual esophagus may be repeated as many times as necessary until an optimum extension of the distal end of the residual esophagus is achieved. In the alternative, the residual esophagus may be extended along its longitudinal axis as many times as necessary to induce a permanent extension along its longitudinal axis. For example the internal pressure of device 10 may, repeatedly, be reduced, and then, increased in order to induce a permanent extension along the longitudinal axis of the residual esophagus.

The present invention may be embodied in other forms without departing from the spirit and the essential attributes thereof, and, accordingly, reference should be made to the appended claims, rather than to the foregoing specification, as indicated the scope of the invention.

## Claims

1. A device for extending a distal end of an anatomic tube comprising:
an accordion pleated bladder, said bladder having a proximal end and a distal end.

2. A device according to Claim 1, wherein said bladder being a biocompatible elastomeric material selected from the group consisting of silicone rubber, natural rubber, styrene-butadiene copolymer, polychloroprene, nitrile rubber, butyl rubber, polysulfide rubbercis-i,4-polyisoprene, ethylene-propylene terpolymers (EPDM rubber), metallocene, and polyurethane rubber.

3. A device according to Claim 1, wherein said bladder further including a small diameter rib or a large diameter rib.

4. A device according to Claim 3, wherein said small diameter rib or large diameter rib having an annular shape.

5. A device according to Claim 3, wherein said small diameter rib or large diameter rib being an integrated component of said bladder or a separate component secured to said bladder.

6. A device according to Claim 1, wherein said proximal end being adapted to expand circumferentially in the range of about 1% to about 30% thereby securing said bladder to a proximal end of an anatomic tube.

7. A device according to Claim 1, wherein said distal end being adapted to expand circumferentially in the range of about 1% to about 30% thereby securing said bladder to a distal end of an anatomic tube.

8. A device according to Claim 1, wherein said proximal end further including means for securing said bladder to a proximal end of an anatomic tube.

9. A device according to Claim 8, wherein said means for securing said bladder to a proximal end of an anatomic tube being a first anchor.

10. A device according to Claim 1, wherein said distal end further including means for securing said bladder to a distal end of an anatomic tube.

11. A device according to Claim 10, wherein said means for securing said bladder to a distal end of an anatomic tube being a second anchor.

12. A device according to Claim 1, wherein said proximal end further including means for connecting said bladder to a pressure generator.

13. A device according to Claim 12, wherein said device further including a pressure generator.

14. A device according to Claim 1, wherein said proximal end having a sealable opening and said distal end being sealed.

15. A method for extending a distal end of an anatomic tube comprising the steps of:
providing a device for extending a distal end of an anatomic tube comprising an accordion pleated bladder, wherein said bladder having a proximal end and a distal end;
placing said device in said anatomic tube;
connecting said device to a pressure generator;
increasing the internal pressure of said device via said pressure generator by pumping a fluid into said device thereby extending said bladder along its longitudinal axis; and
thereby extending said anatomic tube along its longitudinal axis.

16. The method according to Claim 15, wherein said method further comprising the step of:
maintaining said anatomic tube in an extended state for a period of about 1 month to about 6 months thereby inducing a permanent longitudinal extension in said anatomic tube.

17. The method according to Claim 16, wherein said maintaining step further comprising the steps of:
measuring the internal pressure of said device;
pumping more of said fluid into said device thereby extending said extended bladder further along its longitudinal axis; and
thereby extending said anatomic tube further along its longitudinal axis.

18. The method according to Claim 14, wherein said method further comprising the step of:
reducing said internal pressure;
increasing said internal pressure; and
repeating said above steps as many times as necessary to induce a permanent longitudinal extension in said anatomic tube.
